# EUROPEAN PATENT SPECIFICATION

(11) **EP 1 832 261 B1**
(45) Date of publication and mention of the grant of the patent: **27.02.2013**
(21) Application number: 07004961.4
(22) Date of filing: 09.03.2007
(51) Int. Cl.: A61F 13/02

(54) **Pressure-sensitive adhesive tape or sheet**
Druckempfindliches Klebeband oder -folie
Bande ou feuille adhésive sensible à la pression

(30) Priority: 10.03.2006 JP 2006065780; 13.04.2006 JP 2006111389; 13.06.2006 JP 2006163421
(43) Date of publication of application: 12.09.2007
(73) Proprietor: NITTO DENKO CORPORATION, Osaka (JP)
(72) Inventor: Furumori, Kenji, Ibaraki-shi Osaka (JP); Hatanaka, Hiroshi, Ibaraki-shi Osaka (JP); Kasahara, Tsuyoshi, Ibaraki-shi Osaka (JP); Ishikawa, Tatsumi, Ibaraki-shi Osaka (JP); Kikuchi, Shunetsu, Ibaraki-shi Osaka (JP); Ota, Tomoyuki, Ibaraki-shi Osaka (JP)
(74) Representative: Grünecker, Kinkeldey, Stockmair & Schwanhäusser

(56) References cited:
- WO-A1-00/78883
- US-A- 3 085 024
- US-A- 4 581 087
- US-A1- 2004 133 136

## Description

The present invention relates to a pressure-sensitive adhesive tape or sheet and particularly, the present inventions relates to a medical pressure-sensitive adhesive tape or sheet having a hand tearing property and causing less skin irritation, which can be used for a medical care or in the field of hygiene and sports.

A pressure-sensitive adhesive tape or sheet according to the preamble of claim 1 and a pressure-sensitive adhesive tape or sheet according to the preamble of claim 10 is already known from US 3 085 024 A.

The medical pressure-sensitive adhesive tape or sheet (hereinbelow, may also be referred to as a pressure-sensitive adhesive tape) generally has a structure formed with a substrate on which a pressure-sensitive adhesive layer is formed on one side, and there are many pressure-sensitive adhesive tapes or sheets using a nonwoven fabric as the substrate. Pressure-sensitive adhesive tapes formed with such substrate made of nonwoven fabric are economical, and ones having excellent properties such as mechanical strength, adhesivity, and the like, are also found. For the medical pressure-sensitive adhesive tape, there are many cases requiring a hand-cut property to be cut by hand. There have been proposed various reports on the pressure-sensitive adhesive tape having an improved hand-cut property from the past.

JP-B-3431628 discloses a nonwoven sheet including a randomly interlaced fibrous web of tensilized nonfracturable staple fibers and binder fibers or a tape made therefrom, which is formed from pattern embossing techniques. In particular, there proposed a nonwoven sheet material, in which the nonwoven sheet or the tape made therefrom is pattern embossed, and is uniformly interbonded by a chemical bonding agent in whole, and further in which the nonwoven sheet material exhibits a Hand measurement of 250 g or less for a 20 cm square sheet, and is readily finger-tearable in the cross web direction, and also exhibits a machine direction wet and dry-break tensile strength of at least 10 N/cm.

JP-T-9-502111 discloses rolls of perforated nonwoven surgical tape. In particular, there proposed a roll of pressure-sensitive adhesive tape including a binder-containing nonwoven web having a longitudinal axis and a lateral axis which is coated on a major surface with a pressure-sensitive adhesive, in which the adhesively-coated web has a plurality of longitudinally spaced, laterally extending, perforated separation lines defined by a series of about 0.2 to 5 mm perforations separated by about 0.1 to 1 mm connecting segments of the tape and a ratio of perforation length to connecting segment length of about 1:1 to 10:1.

JP-A-2002-360625 discloses a skin sticking pressure-sensitive adhesive tape excellent in an external appearance after cutting by hand, and its manufacturing method. Herein, the support is a polyolefin nonwoven fabric, a large number of penetrating or non-penetrating micropores are formed in the polyolefin nonwoven fabric, and nonwoven fabric fiber of the respective micropores melts to become a film shape.

JP-T-2003-503538 discloses pressure-sensitive adhesive articles formed with fibers and formed from a combination of interbonding, smooth roll calendering, and pattern embossing techniques. In particular, there proposed a pressure-sensitive adhesive article having a nonwoven backing which includes a first surface and a second surface, and a pressure-sensitive adhesive coated on the first surface of the backing. Herein, the backing has an embossed pattern on a fibrous web, and the embossed pattern is selected from the group consisting of at least two rows of a plurality of depressions in a first direction aligned to form columns of the plurality of depressions in a second direction, in which a distance between two depressions in one column varies from a distance between two depressions in a second column and at least two rows of a plurality of depressions in a first direction aligned to form columns of the plurality of depressions in a second direction, and a distance between two depressions in at least one column varies along the first direction.

JP-UM-A-47-18996 discloses a medical pressure-sensitive adhesive tape provided with pores. Herein, there proposed a medical pressure-sensitive adhesive tape formed on a substrate such as a synthetic resin, paper, and nonwoven fabric, with pores having a shape in which the length of width direction is longer than the length of longitudinal direction, and a directivity where the end part in the torn direction is usually sharply-angled.

JP-T-2002-526667 discloses laminate composite materials formed with a combination of a nonwoven fabric, a scrim which is in contact with a filament stretched in the length and cross direction, and a binder.

JP-UM-A-7-38153 discloses a perforated tape. For example, sellotape (registered trademark) or a medical pressure-sensitive adhesive plaster is perforated by the length of 3 cm, 5 cm, or 8 cm, to be readily cut for a handy daily-life use.

In general, a nonwoven adhesive sheet is difficult to be cut by hand or is cut giving the unevenly cut surface in many cases. Although there are many proposals about the pressure-sensitive adhesive tape having a hand tearing property, there are no examples closely examining the state of cut surface when cut by hand. It is ideal that a tape is cut in linear as in the plastic film treated with perforation, and the cut surface is fine, but no such adhesive tape satisfying the requirements has been obtained yet.

In addition, it is necessary that the pressure-sensitive adhesive sheet less causes a skin irritation so as to be used for a medical use.

Further, it is necessary to appropriately adjust the size and distance of penetrating pores so as to obtain the strength of pressure-sensitive adhesive sheet per se. The known pressure-sensitive adhesive sheet is designed for the pitch of perforation for a length direction to be in a regular interval so as to obtain the strength of adhesive sheet per se.

However, it is desirable that the pressure-sensitive adhesive tape is cut in a desired length depending upon a use and circumstances of a user in a practical use regardless of the pitch interval of perforation provided.
Patent Document 1: JP-B-3431628
Patent Document 2: JP-T-9-502111
Patent Document 3: JP-A-2002-360625
Patent Document 4: JP-T-2003-503538
Patent Document 5: JP-UM-A-47-18996
Patent Document 6: JP-T-2002-526667
Patent Document 7: JP-UM-A-7-38153

The present invention is made to solve the above problems, and is aimed to provide a pressure-sensitive adhesive tape or sheet having an excellent one-way hand tearing property.

In addition, the invention is aimed to provide a pressure-sensitive adhesive tape or sheet which can be cut in one direction by hand giving an evenly cut surface.

In addition, the another object of the invention is to provide a pressure-sensitive adhesive tape or sheet which can be cut in a desired length by hand and cut in linear.

The above and further objects of the invention are achieved by a pressure-sensitive adhesive tape or sheet according to claim 1 and a pressure-sensitive adhesive tape or sheet according to claim 10. Preferred embodiments are claimed in the dependent claims.

The present inventors have examined extensively, and as a result, they have found that the object can be achieved by forming a specific pore with the use of a specific substrate, and thus completed the invention.

Fig. 1A is a drawing showing an example of the penetrating pore pattern in the pressure-sensitive adhesive tape or sheet of the present invention.

Fig. 1B is a drawing showing another example of the penetrating pore pattern in the pressure-sensitive adhesive tape or sheet of the invention.

Fig. 2A is a drawing showing an example of the penetrating pore shape in the pressure-sensitive adhesive tape or sheet of the invention.

Fig. 2B is a drawing showing another example of the penetrating pore shape in the pressure-sensitive adhesive tape or sheet of the invention.

Fig. 2C is a drawing showing still another example of the penetrating pore shape in the pressure-sensitive adhesive tape or sheet of the invention.

Fig. 2D is a drawing showing further another example of the penetrating pore shape in the pressure-sensitive adhesive tape or sheet of the invention.

Fig. 3 is a drawing showing the method of measuring a longest length (P) up to the cut edge for evaluating the hand tearing property.

Fig. 4A is a drawing schematically showing a penetrating pore included in the pressure-sensitive adhesive tape or sheet of the invention.

Fig. 4B is a drawing schematically showing another penetrating pore included in the pressure-sensitive adhesive tape or sheet of the invention.

Fig. 4C is a drawing schematically showing a non-penetrating pore included in the pressure-sensitive adhesive tape or sheet of the invention.

Fig. 5 is a drawing schematically showing a projection form included in punched rolls when adhesive tapes prepared in the Examples and Comparative Examples are punched.

Fig. 6 is a drawing schematically showing a sheet cut surface with regard to a hand tearing property test of the Examples.

Fig. 7 is a photograph showing pores included in the pressure-sensitive adhesive sheet. (Example 4)

Fig. 8 is a photograph showing pores included in the pressure-sensitive adhesive sheet. (Comparative Example 4).

### Description of Symbols

- X: length width of penetrating pore
- Y: cross width of penetrating pore
- M: distance of non-cut section in a longitudinal direction
- N: distance of non-cut section in a width direction
- P: maximum length measured with digital caliper
- A: pressure-sensitive adhesive tape
- 1: pore
- 2: penetrated part of pore (hole)
- 3: non-penetrated part of pore (depression)
- 4: interval between pores in cross direction
- 5: cross width of pore
- 6: interval between pores in length direction
- 7: length width of pore
- 8: ideal cut surface
- 9: distance between a cut line of the ideal cut surface and a most distanced cut edge

The present invention relates to the following (1) to (20).
(1) A pressure-sensitive adhesive tape or sheet comprising:
   a substrate comprising a nonwoven fabric; and
   a pressure-sensitive adhesive layer disposed on at least one side of the substrate,
   said pressure-sensitive adhesive tape or sheet having a plurality of penetrating pores which penetrate the substrate and the pressure-sensitive adhesive layer so that corners of said penetrating pores are aligned in at least one direction,
   said penetrating pores having a flat polygonal shape, and
   wherein said penetration pore has, among the corners aligned in one direction, at least two corners facing each other in one direction each having an angle of less than 90°. (Hereinafter, it may be referred as the first embodiment of the invention.)
(2) The pressure-sensitive adhesive tape or sheet according to (1), wherein the flat polygonal shape of said penetrating pore is a flat hexagonal shape or a flat transformed-hexagonal shape.
(3) The pressure-sensitive adhesive tape or sheet according to (1), wherein said penetrating pore is formed by punching with an abacus bead-shaped metal mold.
(4) The pressure-sensitive adhesive tape or sheet according to (1), wherein a distance between the corner of one penetrating pore having an angle of less than 90° and the corner of another penetrating pore adjacent thereto having an angle of less than 90°, said penetrating pores being aligned, is in the range of 0.3 mm to 3 mm.
(5) The pressure-sensitive adhesive tape or sheet according to (1), wherein a distance between one penetrating pore and another penetrating pore adjacent thereto, said penetrating pores being aligned, is equal to or longer than a length of a flattened part of the penetrating pore.
(6) The pressure-sensitive adhesive tape or sheet according to (1), wherein the nonwoven fabric is formed by a thermoplastic resin.
(7) The pressure-sensitive adhesive tape or sheet according to (1), wherein the substrate comprises a binder impregnated therein.
(8) The pressure-sensitive adhesive tape or sheet according to (1), which is subjected to a peeling treatment on the side of the substrate opposite to the side on which the pressure-sensitive adhesive layer is disposed.
(9) The pressure-sensitive adhesive tape or sheet according to (1), which is wound in a roll form for keeping.
(10) A pressure-sensitive adhesive tape or sheet comprising:
   a substrate comprising a nonwoven fabric; and
   a pressure-sensitive adhesive layer disposed on at least one side of the substrate,
   said pressure-sensitive adhesive tape or sheet having a plurality of pores aligned in at least one direction,
   said pores having a shape in which at least two corners facing each other in one direction each has an angle of less than 90°,
   said pores including penetrating and non-penetrating pores,
   said pressure-sensitive adhesive tape or sheet having a penetration ratio of a series of adjacent pores of 50% or more and less than 100%, and
   wherein the penetrating and non-penetrating pores are disposed randomly. (Hereinafter, it may be referred as the second embodiment of the invention.)
(11) The pressure-sensitive adhesive tape or sheet according to (10), wherein a distance between one pore and another pore adjacent thereto among the pores aligned in at least one direction is in the range of 0.3 to 0.75 mm.
(12) The pressure-sensitive adhesive tape or sheet according to (10), wherein the pore has a length of long-sided part in the range of 0.3 to 0.75 mm.
(13) The pressure-sensitive adhesive tape or sheet according to (10), wherein the penetrating pore has a size in the range of 1 × 10⁻⁴ to 3 mm².
(14) The pressure-sensitive adhesive tape or sheet according to (10), wherein the nonwoven fabric is formed by a thermoplastic resin.

Hereinbelow, the invention will be described in more detail.

First, the first embodiment of the invention as one example of preferred embodiments of the invention will be described in detail.

The pressure-sensitive adhesive tape or sheet according to the first embodiment of the invention is a pressure-sensitive adhesive tape or sheet comprising:
a substrate comprising a nonwoven fabric; and
a pressure-sensitive adhesive layer disposed on at least one side of the substrate,
said pressure-sensitive adhesive tape or sheet having a plurality of penetrating pores which penetrate the substrate and the pressure-sensitive adhesive layer so that corners of said penetrating pores are aligned in at least one direction,
said penetrating pores having a flat polygonal shape, and
wherein said penetration pore has, among the corners aligned in one direction, at least two corners facing each other in one direction each having an angle of less than 90°.

Herein, the flat polygonal shape of the penetrating pore is preferably a flat hexagonal shape or a flat transformed-hexagonal shape.

In addition, the pressure-sensitive adhesive tape or sheet of the invention comprising a substrate including a nonwoven fablic and a pressure-sensitive adhesive layer disposed on at least one side of the substrate is preferably a pressure-sensitive adhesive tape or sheet in which a plurality of penetrating pores which penetrate the substrate and the pressure-sensitive adhesive layer are disposed so that corners of the penetrating pores are aligned in at least one direction and the penetrating pores are formed by punching with an abacus beads-shaped metal mold.

According to the invention, a distance between the corner of one penetrating pore having an angle of less than 90° and the corner of another penetrating pore adjacent thereto having an angle of less than 90°, the penetrating pores being aligned, is in the range of 0.3 mm to 3 mm.

According to the invention, a distance between one penetrating pore and another penetrating pore adjacent thereto, the penetrating pores being aligned, is equal to or longer than a length of a flattened part of the penetrating pore.

According to the invention, the nonwoven fabric included in the substrate can be formed by a thermoplastic resin.

In addition, the substrate preferably includes a binder impregnated therein.

In the invention, it is preferable that the side of the substrate opposite to the side on which the pressure-sensitive adhesive layer is disposed is subjected to a peeling treatment.

In the invention, the pressure-sensitive adhesive tape or sheet can be wound in a roll for keeping.

According to the invention, a medical pressure-sensitive adhesive sheet having an excellent one-way hand tearing property can be provided.

In the invention, the substrate including nonwoven fabric has penetrating pores. It is necessary that the penetrating pores also penetrate the pressure-sensitive adhesive layer. Thus, the pressure-sensitive adhesive layer may be partially provided avoiding the penetrating pores on a substrate having penetrating pores, or the penetrating pores may be formed after the pressure-sensitive adhesive layer is provided. The penetrating pores will be described later.

In the invention, it is preferable that the medical pressure-sensitive adhesive tape or sheet has penetrating pores and the penetrating pores are provided to be aligned in at least one direction. The pores (penetrating pores) are, for example, provided in a longitudinal direction and/or a cross direction at a regular interval. The pores are in a shape having an angle of less than 90° on at least two places (corners), and the two places with the angle of less than 90° are present in at least one direction of longitudinal direction and cross direction of nonwoven fabric. In the invention, the two places are preferably present at least in the cross direction. In addition, corners of the two places are preferably placed to be substantially aligned in a straight line. The shape of the penetrating pore is a flat polygon, which has the bulged center part and corners having an angle formed when two sides are met of less than 90° at the right and left parts. For example, the shape of the penetrating pore includes polygons such as a flat hexagonal shape (including an abacus-beads shape), a flat transformed hexagonal shape i.e., the shape where the bulged center part is poly-angulated or curved, or the sharp corner is formed from two concave curves, and an octagonal shape.

It is preferable for the size of the penetrating pore to be appropriately set, but preferable that the area of penetrating pore is in the range of 0.09 to 3 mm². When the pore area is less than 0.09 mm², the air permeability may not be sufficiently improved or a comfortable hand tearing property may not be obtained. Meanwhile, when the pore area is more than 3 mm², the strength of sheet to be obtained may deteriorate or a sufficient adhesivity may not be obtained. It is necessary to have penetrating pores in the invention, but a case where some are not penetrated may also be included in the technical range of the invention so long as the most are penetrated and it is within the scope of not impairing the purpose of the invention.

With regard to the distance between the penetrating pores to be provided, it is preferable that the distance between the corner of one penetrating pore and the corner of another penetrating pore adjacent thereto is 0.3 mm or more and 3 mm or less for the distance between non-cut sections in a cross direction and is 1 mm or more and 10 mm or less for the distance between non-cut sections in a longitudinal direction. When the distance of adjacent pores in a cross direction is less than 0.3 mm, there may be a case where the sufficient strength is not provided to a sheet to be obtained, and when it is more than 3 mm, there may be a case where the air permeability cannot be improved or the hand tearing property cannot be attained. The distances in the longitudinal direction and the cross direction are not necessarily the same, and for example, the distance in a longitudinal direction may be set longer than the distance in a cross direction to surely provide a hand tearing property of a cross direction to a nonwoven fabric. In the invention, the longitudinal direction indicates the machine direction of the sheet and the cross direction indicates the width direction of the sheet.

In the invention, it is preferable that the cut section is partly formed between a tip of sharp corner of a penetrating pore and a tip of sharp corner of an adjacent penetrating pore on a nonwoven fabric. Forming the cut section can more surely provide a hand tearing property to the pressure-sensitive adhesive tape or sheet. It is preferable that the shape and size of the cut section, and its place to be positioned are designed in consideration of the balance of entire strength for the sheet or the like. Such cut section may be formed in the process of forming a nonwoven fabric or may be formed after forming a nonwoven fabric.

In the invention, the penetrating pores may be formed after forming the pressure-sensitive adhesive layer on a substrate, or the penetrating pores may be formed first and then the pressure-sensitive adhesive layer may be formed on a substrate. In the former case, penetrating pores penetrating the substrate and the pressure-sensitive adhesive layer can be formed with the use of a metal roll on the substrate having a pressure-sensitive adhesive layer. In the latter case, penetrating pores are formed in accordance with the above method on a substrate treated with a chemical binder according to the necessity, and then a pressure-sensitive adhesive layer is provided so that the penetrating pores are not blocked. For example, the pressure-sensitive adhesive layer may be formed in a striped form avoiding penetrating pores on a substrate or the pressure-sensitive adhesive layer may be partly formed.

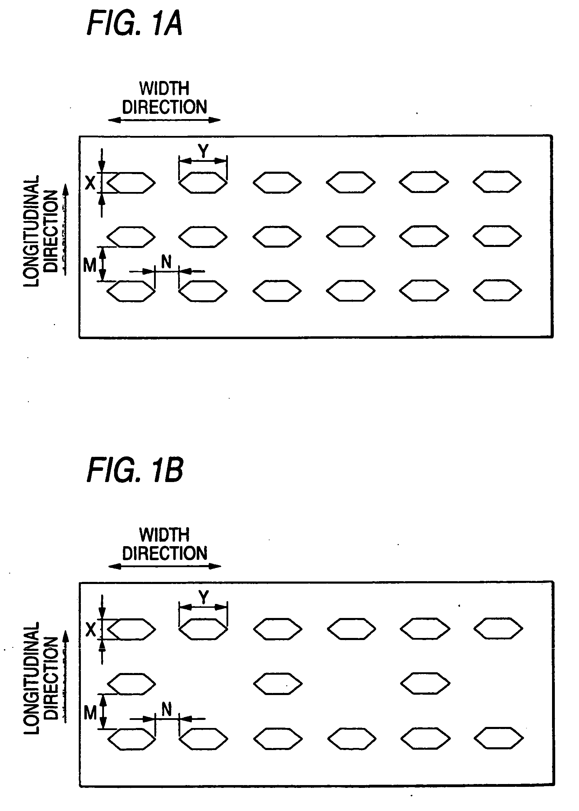

A penetrating pattern in the pressure-sensitive adhesive tape or sheet of the invention will be described in more detail with reference to the accompanying drawings. Fig. 1A is a drawing showing an example of the penetrating pore pattern. Here, penetrating pores having a flat hexagonal shape are aligned at a constant interval across the length and cross. In addition, the penetrating pores in Fig. 1A are disposed so that the sharp corners of the pores are aligned in a cross direction (width direction), and thus provides an excellent cross-direction hand tearing property.

It is preferable that a distance (M) of non-cut section which is the distance between penetrating pores adjacent to each other in a longitudinal direction for a column alignment (machine direction of nonwoven fabric, longitudinal direction), and a distance (N) of non-cut section which is the distance between penetrating pores adjacent to each other in a cross direction for a cross alignment (direction perpendicular to the machine direction, width direction), are appropriately determined depending upon the shape and size of the penetrating pores and the alignment pattern of the penetrating pores. For example, when the penetrating pores each having a flat hexagonal shape are aligned as in the Fig. 1A, the distance (M) of non-cut section in a longitudinal direction is preferably in the range of 1 to 10 mm, and the distance (N) of non-cut section which is the distance between penetrating pores adjacent to each other in a cross direction for a cross alignment (direction perpendicular to the machine direction, width direction) requiring a hand tearing property, in other words the distance between sharp corners of adjacent penetrating pores, is preferably in the range of 0.3 to 3 mm. The length width of a penetrating pore (X) is preferably in the range of 0.1 to 1 mm, and the cross width (Y) is preferably in the range of 0.2 to 2 mm.

Fig. 1B is a drawing showing another example of the penetrating pore pattern, which shows the state where penetrating pores shown in the Fig. 1A are partly filled, that is, the state where flat hexagonal shapes are partly missed, and shows that the distance between penetrating pores aligned in lengthwise and crosswise is not constant. The pressure-sensitive adhesive tape or sheet shown in Fig. 1B has a deteriorated hand tearing property in the center of cross alignment, but the upper array and the lower array of cross alignment have hand tearing property. The distance (N) between penetrating pores adjacent to each other in a cross direction for a cross alignment (width direction) requiring a hand tearing property is preferably in the range of 0.3 to 3 mm. The distance (M) of non-cut section in a longitudinal direction is preferably in the range of 1 to 10 mm. The length width of penetrating pore (X) is preferably in the range of 0.1 to 1 mm, and the cross width (Y) is preferably in the range of 0.2 to 2 mm.

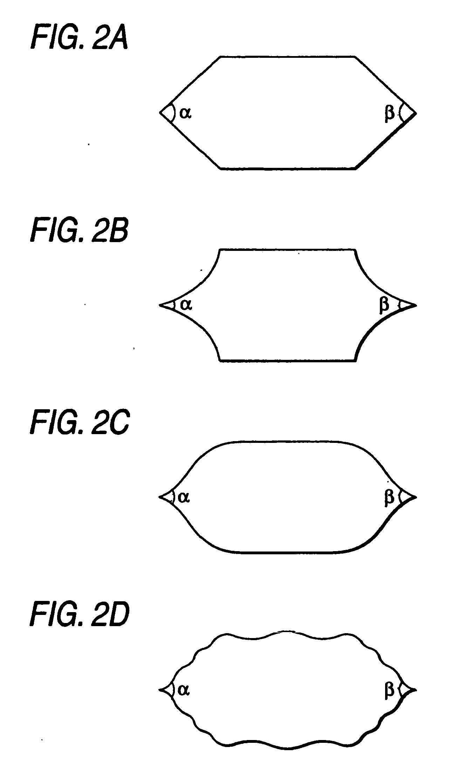

Specific examples of the penetrating pore shape are shown in Figs. 2A to 2D. Fig. 2A shows a penetrating pore having a flat hexagonal shape. Fig. 2B shows a penetrating pore having a flat transformed-hexagonal shape, in which the two sides forming sharp corners of the flat hexagon are each formed in a concaved curve and the angles of sharp corners are narrower than the angles in the Fig. 2A. Fig. 2C shows a penetrating pore having a flat transformed-hexagonal shape, in which the upper and the lower parts of the hexagon are each formed in a curved line and the sharp corners are formed with concaved curves. Fig. 2D shows a penetrating pore having a flat transformed-hexagonal shape, which is substantially a flat hexagonal shape but angles are not definitely formed. For the flat transformed-hexagonal shape in the invention, such mentioned shapes may also be included. Herein, only the hexagonal shapes are represented, but octagonal shapes, decagonal shapes, dodecagonal shapes, and the like may also be included without being limited by the hexagonal shapes, provided that the two tip angles (α, β) facing each other are essentially the acute angle.

The invention having penetrating pores with a flat hexagonal shape is exemplified in Figs. 1A and 1B, but the invention having one of penetrating pore shapes shown in Figs. 2A to 2D or a flat polygonal shape may also be included. In this case, there may be formed a penetrating pores having other shape such as a flat transformed-hexagonal shape or the like instead of the flat hexagonal shape.

The penetrating pores in the invention can be formed with the use of an abacus beads-shaped metal mold as mentioned above, and the penetrating pores formed in such manner are included in the range of penetrating pores in the invention.

Subsequently, the second embodiment of the invention as another preferred embodiment of the invention will be described in detail.

The pressure-sensitive adhesive tape or sheet related to the second embodiment of the invention is a pressure-sensitive adhesive tape or sheet comprising:
a substrate comprising a nonwoven fabric; and
a pressure-sensitive adhesive layer disposed on at least one side of the substrate,
said pressure-sensitive adhesive tape or sheet having a plurality of pores aligned in at least one direction,
said pores having a shape in which at least two corners facing each other in one direction each has an angle of less than 90°,
said pores including penetrating and non-penetrating pores,
said pressure-sensitive adhesive tape or sheet having a penetration ratio of a series of adjacent pores of 50% or more and less than 100%, and
wherein the penetrating and non-penetrating pores are disposed randomly.

Here, it is preferable that the distance between one pore and another pore adjacent thereto among the pores aligned in at least one direction is in the range of 0.3 to 0.75 mm.

The length of long-sided part of the pore is preferably in the range of 0.3 to 0.75 mm.

The size of the penetrating pore is preferably in the range of 1 x 10⁻⁴ to 3 mm².

The nonwoven fabric is preferably formed by a thermoplastic resin.

According to the invention, the pressure-sensitive adhesive tape or sheet having an excellent hand tearing property can be obtained. Further, since the pressure-sensitive adhesive tape or sheet of the invention includes a substrate including a nonwoven fabric and a pressure-sensitive adhesive layer disposed on at least one side of the substrate, it provides a pleasant texture. In particular, since the pressure-sensitive adhesive tape or sheet of the invention has penetrating pores, it provides an excellent hand tearing property and is an adhesive tape or sheet which does not cause dampness and irritation and is kind for skin.

The pressure-sensitive adhesive tape or sheet of the invention has a substrate including a nonwoven fabric and a pressure-sensitive adhesive layer disposed on at least one side of the substrate. The pressure-sensitive adhesive tape or sheet is provided with pores aligned in at least one direction. In the invention, the pores include penetrating and non-penetrating pores. For the pores aligned in one direction, the penetrating and non-penetrating pores may be aligned in a random order.

In the invention, the term "aligned in at least one direction" means the alignment in one direction or a plural directions. The pore alignment can be appropriately set in accordance with the configuration of the pressure-sensitive adhesive tape or sheet. For example, when the pressure-sensitive adhesive tape or sheet is in a roll form, the pores can be aligned parallel to the roll width direction of the pressure-sensitive adhesive tape in a regular interval. When the pressure-sensitive adhesive tape or sheet is in a sheet form, the pores can be aligned across the length and cross directions in a regular interval. The distance between the adjacent alignments of pores can be appropriately determined in accordance with the size and use purpose of the pressure-sensitive adhesive tape. For example, when the rolled pressure-sensitive adhesive tape is for a medical use, the distance can be set in the range of 3 to 10 mm so that the tape is cut in a desired length.

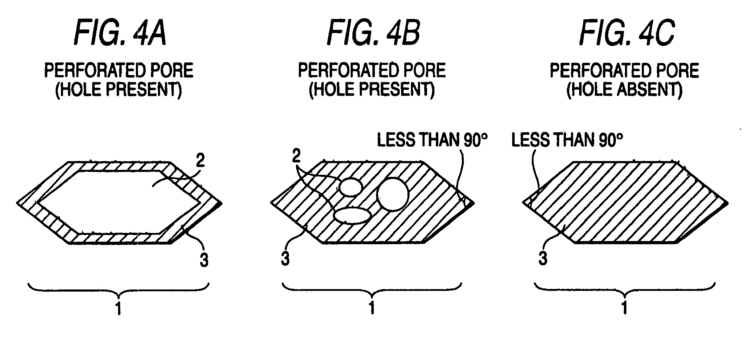

In the invention, the pore has a shape in which at least two corners facing each other in one direction each have an angle of less than 90°, as shown in Figs. 4A to 4C. When the pore has a shape in which the corners facing each other in one direction each have an angle of more than 90°, there may be a case where the length of the one direction side becomes equal or less as compared to the length of side perpendicular to the one direction side. In such a case, although the pores having a shape in which the corners facing each other in one direction each have an angle of more than 90° are aligned in one direction, it is hard to say that the excellent hand tearing property can be exhibited.

In the invention, for the size of pores, the area is preferably in the range of 0.09 to 3 mm². When the pore area is less than 0.09 mm², the pemetration reduces in size, thus becomes hard to be cut in one direction by hand. When the pore area is more than 3 mm², the strength necessary in the case of using as a medical tape cannot be obtained.

In the invention, for the interval between pores to be positioned in one direction, the distance between pores adjacent to each other is preferably in the range of 0.3 to 0.75 mm. When the distance is shorter than 0.3 mm, a sufficient strength cannot be obtained and a sufficient adhesivity cannot be obtained. When the distance is longer than 0.75 mm, a sufficient permeability cannot be obtained and a sufficient hand tearing property cannot be obtained.

In the invention, when the pore is formed by a punch roll (metal roll) which will be described later, the projection of the punch roll has a shape in which corners facing each other in one direction each have an angle of less than 90°, and for example, is in a polygonal shape in which the length of one direction side is longer than the length of a side perpendicular to the one direction side, and specifically can be mentioned by the form of an abacus beads of hexagonal shape.

The pores formed in accordance with the above method include penetrating and non-penetrating pores. In the invention, the term "penetrating pore" represents the pore at least partly having a penetration as shown in Figs 4A and 4B. As shown in Fig. 4A, the penetrating pore shape is almost the same to that of the pore, and may be a shape in which corners facing each other in one direction each have an angle of less than 90°, or as shown in Fig. 4B, the penetrating pore partly having the penetration may be any shape so long as it is penetrated. In the invention, the term "non-penetrating pore" represents the case of forming a film in the non-penetrated form (depression).

With regard the size of the penetrating pore, in the case that the penetrating pore partly has the penetration as shown in Fig. 4B, the penetrating pore area is preferably in the range of 1 x 10⁻⁴ to 3 mm². When the penetrating pore area is smaller than 1 x 10⁻⁴ mm², the penetrating pore is overly small, thus it is difficult to be cut in one direction by hand. When the penetrating pore area is larger than 3 mm², the strength necessary for using as a medical tape cannot be obtained.

In the invention, the term "penetration ratio" represents the ratio of the number of penetrating pores to the sum of the number of penetrating pores and the number of non-penetrating pores. According to the invention, the penetration ratio of a series of adjacent pores is 50% or more and less than 100%. The term "a series of adjacent pores" means the series of pores aligned on a cut surface in the case of cutting the pressure-sensitive adhesive tape or sheet, and for example means a series of pores set in a roll width direction in the case that the pressure-sensitive adhesive tape is in a roll form. When the penetration ratio is 0% or more and less than 50%, a sufficient hand tearing property cannot be obtained because the penetrating pores necessary for providing a hand tearing property are either not present or less present.

Subsequently, an example will be described in detail, which does not form part of the invention but is useful for the understanding of the invention.

The pressure-sensitive adhesive tape or sheet according to the example is a pressure-sensitive adhesive tape or sheet comprising:
a substrate comprising nonwoven fabric; and
a pressure-sensitive adhesive layer disposed on at least one side of the substrate,
said pressure-sensitive adhesive tape or sheet having a plurality of pores so that edges of said pores are aligned in at least one direction,
wherein a distance between one alignment of said pores and another alignment of said pores adjacent thereto is in the range of 1 to 15 mm.

The nonwoven fabric can be formed by a thermoplastic resin.

In the invention the substrate may include a binder impregnated therein.

In the invention, the side of the substrate opposite to the side on which the pressure-sensitive adhesive layer is disposed may preferably be subjected to a peeling treatment.

In the invention, the pressure-sensitive adhesive tape or sheet can be wound in a roll for keeping.

According to the invention, a medical pressure-sensitive adhesive tape or sheet having an excellent hand tearing property can be obtained.

The substrate included in the pressure-sensitive adhesive tape of the invention has pores. The pores are disposed so that the edges of the pores are aligned in at least one direction. Here, the term "edge" refers to a corner, one side, or a part of arc. The shape or size of the pore is not particularly limited, but the pore shape, for example, can be arbitrary selected from a linear shape, a round shape, a trapezoidal shape, a box shape, a polygonal shape, and the like. Here, it is necessary that the pores are disposed so as to apparently form an alignment when seen as a whole. For example, when the pore is in a linear shape, the pores are oriented for the longitudinal direction in a linear form to be in an alignment, when the pore is in a circular shape, the pores are oriented to form an alignment when the circular pores are linked, and when the pore is in a box shape, the pores are oriented for the angle tips to be in an alignment.

With regard to the distance between the alignments of pores constituting the pored parts, the distance between an alignment and another alignment adjacent thereto is in the range of 1 to 15 mm, preferably 1 to 10 mm, and more preferably 3 to 7 mm. When the pores are formed so that the distance between an alignment and another alignment adjacent thereto is 1 mm or more, straight cut surface can be achieved even by the cutting by hand. That is, it is possible to cut the tape along one alignment of pores without cutting across the adjacent alignment. In addition, when the distance is 15 mm or less, it is possible to cut the tape in a desired length, or in a predetermined length depending upon the use, or in a desired length determined by circumstances of a person using the pressure-sensitive adhesive tape. When the distance is more than 15 mm, the length of the pressure-sensitive adhesive tape has to be increased or reduced than the desired length to be cut, thus has a problem in usability, and also when the cutting is forcibly done for a desired length, the straight cutting may not be achieved and readily gives an unevenly cut surface.

The pore may be a penetrating pore or non-penetrating pore in a depression state, and also may be in combination thereof so long as not giving an adverse effect on a hand tearing property. From the viewpoint of hand tearing property, it goes without saying that all the pores are preferably penetrating pores.

The pressure-sensitive adhesive tape or sheet of the invention has a pressure-sensitive adhesive layer disposed on at least one side of a substrate. The substrate includes a nonwoven fabric. The nonwoven fabric to be used in the invention may be chemical synthetic fibers or fibers other than those, and the fiber length and size are not particularly limited. Examples of the nonwoven fabric include natural fibers such as cotton, hemp, and wool, regenerated fiber such as rayon and cupra, half synthetic fibers such as acetate and promix, synthetic fibers such as nylon, polyester, acrylic based fiber, vinylon, polyvinyl chloride, vinylidene, polyolefin based fiber, polyurethane chlor, fluorocarbons based fiber, and novoloid based fiber, inorganic fibers such as glass fibers, carbon fibers, alumina fibers, silicon carbide fibers, slag fibers, metal fibers, and wood pulp. The nonwoven fabric for the invention is preferably formed by a thermoplastic resin, and polyolefin-based fibers and polyester-based fibers are preferably used from the viewpoints of availabilities and stability. The nonwoven fabric may be formed with the use of two or more materials or may be formed in a laminate including two or more different layers.

The method of forming the nonwoven fabric is not particularly limited, and a common process for producing a nonwoven fabric can be employed. Examples include dry processes such as adhesive agent type e.g., immersing process, printing process, spraying process, powdering process, thermal bonding process, etc., mechanical bonding type e.g., felting process, stitching process, needle punching process, etc., hydroentanglement type e.g., spunlace, spinning type e.g., spunbonding process, netting process, melt blowing process, filming process, etc.; and wet processes such as hydroentanglement type e.g., spunlace, spinning type e.g., spunbonding process, flash process, and paper making process e.g., thermal-adhesive fabricating process, thermal-compression bonding process, adhesive process, and the like. In the invention, two or more kinds of nonwoven fabrics obtained in accordance with the different forming processes may be laminated.

For the nonwoven fabric constituting the substrate, the basis weight of the total nonwoven fabric is preferably in the range of 10 g/m² to 80 g/m². When a texture and a mechanical strength are considered, the basis weight of the total nonwoven fabric is more preferably in the range of 12 g/m² to 60 g/m².

Pores are formed on the nonwoven fabric constituting a substrate of the pressure-sensitive adhesive tape or sheet of the invention. According to the invention, it is preferable that the nonwoven fabric is subjected to a chemical binding process before forming the pores on the nonwoven fabric. For example, the chemical binding process can be conducted by immersing the nonwoven fabric to a chemical binder or by coating at least one surface of the nonwoven fabric with the coating solution for a chemical binding.

The chemical binder is not particularly limited and a common chemical binder can be used. However, it is preferable that the chemical binder causes less chemical skin irritation, that the chemical binder brings flexibility to avoid giving a discomfort when the chemical binding-treated nonwoven fabric comes in contact with skin, and that the chemical binding-treated nonwoven fabric exhibits moisture permeability after the chemical binder is applied.

Preferred examples of the chemical binder used in the invention include an acryl resin, a vinyl acryl resin, acetate/ethylene, polyvinyl acetate, and the like. Examples thereof further include acryl resin-conjugated latexes, styrene/butadiene rubbers, vinyl acetate/ethylene, vinyl acetate/acrylates, polyvinyl chloride, polyvinyl alcohols, polyurethanes, vinyl acetates, acryl/vinyl acetates, and the like, and further include water-based chemical binder similar to those such as acrylic latex binder, styrene/butadiene rubber latex, acrylic/ vinyl acetate copolymer latex, and the like.

The nonwoven fabric may be subjected to a chemical binding process by coating a water-based chemical binder using a winding rod, a kiss roll, a reverse roll, an air knife, a gravure roll, a spray, or the like.

It is preferable that the used amount of chemical binder is appropriately determined depending upon the desired property for the medical pressure-sensitive adhesive tape i.e., the mechanical strength (drying temperature, wet strength) and the tearing property. In general, the use amount of chemical binder is preferably in the range of about 2 g/m² to about 50 g/m², more preferably in the range of about 3 g/m² to about 50 g/m², and even more preferably in the range of about 5 g/m² to about 35 g/m² .

In the invention, it is preferable that a back surface treatment is carried out after subjecting the nonwoven fabric to the chemical binding process. When the nonwoven fabric is subjected to a back surface treatment, the formed pressure-sensitive adhesive tape or sheet can be smoothly wound back although the pressure-sensitive adhesive sheet is kept in a roll form i.e., formed as a rolled material. For example, the back surface treatment is preferably carried out by coating a back surface treatment agent based on silicon, fluorine, long-chained alkyl, or wax. It is preferable that the use amount of back-surface treatment agent is appropriately selected considering the kind of adhesive agent, and the like.

In the invention, the process for forming the pores is not particularly limited and conventional processes can be appropriately used. The pores, for example, can be formed by die roll cutting by a blade, cutting by laser irradiation, punching by a metal roll, or the like. For example, for forming the pores on a substrate by punching with a metal roll, a substrate such as nonwoven fabric, etc., is placed between the rolls, the metal roll (punch roll) which equips a projection having a pore shape and the pressure roll (metal roll having a smooth surface) which are arranged in an opposed manner, and the substrate is passed through them to form pores on the substrate. In this case, the projections on the metal roll for forming pores are arranged at a constant interval, and a tip end of the projection may be sharp or flat.

As the pressure roll, a stainless roll having a smooth surface or a rubber roll having a smooth surface on which a silicone rubber is coated can be used.

The metal roll is preferably heated to about 150°C or above to about 400°C or below, and the pressure roll is preferably heated to about 50°C or above to 150°C or below. When the metal roll is heated to below 150°C, a thermoplastic resin sheet cannot be melted. When the metal roll is heated to above 400°C, a thermoplastic resin sheet melts, and thus becomes difficult to form penetrating pores by punching.

It is preferable that the pressure loaded between the metal roll and the pressure roll, and the rate (flow rate) of passing the nonwoven fabric through the rolls, are set to form suitable pores on the nonwoven fabric by considering over the projection shape of the metal roll, temperature of the projection, and the thickness of the nonwoven fabric. In general, the pressure between rolls is preferably in the range of about 5 kg/cm to about 200 kg/cm in terms of a linear pressure, and the flow rate for passing a nonwoven fabric is preferably in the range of about 1 m/min to about 50 m/min. When the linear pressure is below 5 kg/cm, a penetrating pore cannot be obtained, and when the linear pressure is above 200 kg/cm, the punch roll may be deformed.

The pressure-sensitive adhesive tape of the invention has a pressure-sensitive adhesive layer on one side of a substrate. For example, a pressure-sensitive adhesive layer is disposed on the side of the nonwoven fabric opposite to the side coated by a chemical binding. As the adhesive agent used for forming the pressure-sensitive adhesive layer, an agent which can be adhered to skin, has flexibility to track the skin, causes less irritation to the skin (chemical irritation, physical irritation), and has moisture permeability, is preferably used, and for example, an adhesive agent for a medical patch can be used. Examples of the adhesive agent satisfying such requirements include a synthetic rubber-based adhesive agent which mainly includes a styrene-isobutylene-styrene copolymer, a polyurethane-based adhesive agent, a polysiloxane-based adhesive agent, a natural rubber-based adhesive agent, a polyether-based adhesive agent, and an acryl-based adhesive agent, where these may be used alone or in combination of two or more kinds.

As an acryl-based polymer forming the acryl-based adhesive agent, acrylic ester is preferably used. Examples of the acrylic ester include acrylic acid or methacrylic acid esters having an aliphatic group such as a butyl group, a 2-ethylhexyl group, an isononyl group, or an isooctyl group. In order to improve the cohesiveness and adhesivity, acrylic acid or methacrylic acid esters may be copolymerized as the vinyl ester having a carboxyl group, according to the necessity.

In the invention, according to the necessity, a mononer having a functional group which may be a crosslinking point to a side chain such as a hydroxyl group, an amino group, and an epoxy group, or a polyfunctional unsaturated monomer having two or more polymerizable carbon-carbon double bonds in one molecule such as divinyl acrylate, trimethylpropanol triacrylate, dipentaerythritol hexa-acrylate, and pentaerythritol triacrylate, can be used in combination as a copolymerizable monomer.

The acryl-based polymer used in the invention may further include an initiating agent. The initiating agent is not particularly limited, and for example, common initiating agents capable of generating a radical by UV irradiation or by thermal decomposition such as peroxides e.g., hydrogen peroxide, benzoyl peroxide, etc., and azo-based compounds typified by azobisisobutylonitrile (AIBN) can be used.

The polymerization method for forming an acryl-based polymer is not particularly limited, and a solution polymerization method, an emulsion polymerization method, a suspension polymerization method, or the like can be used. For example, the polymerization can be conducted by generating a radical using an initiating agent for a thermal decomposition or generating a radical using an initiating agent under a UV irradiation, in the presence of a solvent or water.

The adhesive agent may be optionally blended with various additives such as plasticizer typified by polyalcohols e.g., glycerine, polyethylene glycol, polypropylene glycol, etc., an aqueous or water-absorbent resin e.g., polyacrylic acid, polyacrylic crosslinker, polyvinylpyrrolidone, etc., a tackifier based on rosin, terpene, or petroleum, various flexibilizers, fillers, pigments, and the like.

The pressure-sensitive adhesive layer is formed by using the adhesive agent, and the pressure-sensitive adhesive layer may be provided on an entire substrate or may be partly provided. When the pressure-sensitive adhesive layer is partly provided on a substrate, it may be provided in a dot form or a striped form. It is preferable to have a space functioning for aeration since the skin irritation readily occurs if the skin damps. For the striped form, a space functioning for aeration is preferably provided, and examples include a linear form and a wave form, or further include other forms. In general, a wave form causing less change over time in interrow spaces is preferable. Further, the pressure-sensitive adhesive layer may be a laminate in which two or more layers having different adhesivity are laminated. In this case, one adhesive layer may be provided on an entire substrate and other adhesive layer may be partly provided. It is preferable that the form of adhesive layer is appropriately determined depending upon properties of the pressure-sensitive adhesive layer and a site for the pressure-sensitive adhesive tape or sheet to be used.

The pressure-sensitive adhesive layer may be formed using an adhesive agent by a solution coating, an emulsion coating, or a hot-melt coating.

The pressure-sensitive adhesive layer may be provided before forming pores on the nonwoven fabric, or may be formed after forming pores. When the pressure-sensitive adhesive tape is formed by providing the pressure-sensitive adhesive layer after forming the pores, pores on the pressure-sensitive adhesive tape may be each a penetrated pore or a non-penetrated depression pore, or may be in a state where the penetrated pore is blocked with a pressure-sensitive adhesive layer.

It is preferable that the thickness of the pressure-sensitive adhesive layer constituting the medical pressure-sensitive adhesive tape of the invention is appropriately determined depending upon a site for the medical pressure-sensitive adhesive tape to be applied and properties of the pressure-sensitive adhesive layer. The thickness is, for example, preferably in the range of 10 to 100 µm, and more preferably in the range of 20 to 70 µm. When the pressure-sensitive adhesive layer has the thickness of 10 µm or more, a sufficient adhesivity can be exhibited when attached on the skin. When the thickness is 100 µm or less, a demanded level of vapor permeability for the skin sticking adhesive tap can be obtained, thereby providing a perspiration resistance, and also skin irritation caused by a long term attachment can be avoided.

The pressure-sensitive adhesive layer may be either directly or indirectly provided on the side of the nonwoven fabric opposite to the side treated with a chemical binding,. Herein, the term "indirectly" means that the substrate (including the substrate treated with a chemical binding) and the pressure-sensitive adhesive layer are not directly in contact with each other, and for example, means that the substrate surface is coated with a primer coating agent to improve the anchoring for the pressure-sensitive adhesive layer and the substrate and then the layer formed by coating with the primer coating agent is disposed between the substrate and the pressure-sensitive adhesive layer for a lamination.

The pressure-sensitive adhesive tape of the invention may be covered with a release liner on a surface of the pressure-sensitive adhesive layer before the use to prevent contamination on a surface of the pressure-sensitive adhesive layer. As the release liner, those commonly used for the adhesive tape or sheet to be applied to the skin can be used. In specific, those prepared by coating a releasing agent having a releasing ability such as silicone onto a surface of quality paper, glassine paper, or parchment paper, or those prepared by coating a release agent having a releasing ability such as silicone onto a surface which is anchor coated with a resin on a quality paper or laminated with polyethylene, can be used.

The pressure-sensitive adhesive tape or sheet of the invention may be in a sheet form or in a wound roll form. When it is in a sheet form, a release sheet may be provided on a pressure-sensitive adhesive layer for protecting the pressure-sensitive adhesive layer. The release sheet, in which the side contacting the pressure-sensitive adhesive layer is subjected to a peeling treatment using a silicone-based resin or the like, can be used. When it is in a roll form, a surface treatment is preferably conducted against a support face subjected to the chemical binding. The rolled adhesive tape can be prepared by winding a pressure-sensitive adhesive sheet in a sheet form around a paper core and then cutting by the length of 10 to 100 mm in a rolled width direction. The cut width of 10 mm or less leads to the reason for a difficult handling in practical use, and the cut width of 100 mm or more causes difficulty in sticking.

Since the pressure-sensitive adhesive tape or sheet of the invention has the above-described constitution, it can be torn by hand without using a cutting instrument such as scissors, and also has less irritation to the skin, thereby causing no dampness. Thus, the invention is effective for a medical use.

According to the invention, a medical tape or sheet such as adhesive plasters can be formed using the medical pressure-sensitive adhesive tape. For example, the medical pressure-sensitive adhesive sheet can be cut in an appropriate size to form a plaster or a tape for taping, or form a medical tape or sheet such as dressings covering the wounded area, a protecting material used after a surgical operation, and a covering material e.g., catheter inserting part, gauze, etc., and a medical article such as a fixing tape in which a medical pressure-sensitive adhesive tape is combined with other supporter, a fixing tape for bathing pouch, an instrument holding tape. The invention can be suitably used in a tape for body reinforcement or correction for a body work, or as a tape for sport taping. The medical pressure-sensitive adhesive tape or sheet of the invention particularly exhibits an excellent effect on the hand tearing property, and can be cut in an arbitrary length by hand without giving an uneven cut face, thus obtains a favorable cut face. In addition, it goes without saying that the skin sticking adhesive tape or sheet of the invention can be used for the other purposes than the medical use when it is for the purpose of sticking onto the skin.

### EXAMPLES

The present invention will be described in more detail with reference to examples below, but the invention is not limited by these and various applications are possible within the scope of not impairing the technical idea of the invention.

In the following examples, "parts" indicates "parts by weight" and "%" indicates "% by weight" unless otherwise specified. The measurement techniques and evaluation methods used in the examples will be represented.

First, examples related to the first embodiment of the invention will be described below.

### Tensile Strength Measurement

The tensile strength measurement is carried out by using an autograph "AG-IS" manufactured by Shimazu Corporation. A force applied to the sample having the width of 12 mm until the point of break at the extension rate of 300 mm/min under setting the inter-chuck distance of 100 mm was measured to obtain the value. This measurement operation was repeated 3 times and an average value of the values obtained was determined.

### Hand Tearing Property Evaluation Method

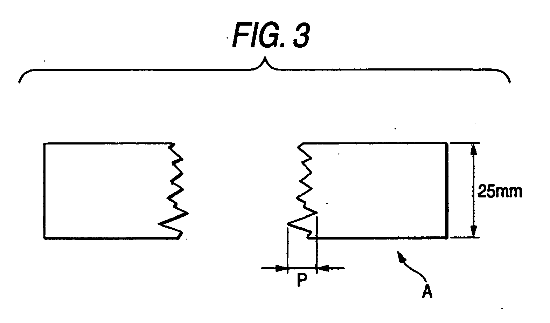

A medical pressure-sensitive adhesive tape was torn with fingers in the length of 25 mm in the direction (width direction) perpendicular to a machine direction. The torn direction was assumed as an ideal cut line, and a distance between the ideal cut line and a most distanced cut edge was obtained. That is, when the tape was torn in a machine direction, a longest length (P) among the edges of the pressure-sensitive adhesive tape stretched in a direction perpendicular to a machine direction was measured. As the length was shorter, the hand tearing property was excellent. The aforementioned operation was repeated for 5 times, and the average value thereof was obtained. The length was measured with the use of Mitutoyo Digital Caliper "CD-20". In Fig. 3, the measurement method of longest length (P) up to the cut edge for the hand tearing property evaluation is shown.

### Example 1

Fifteen g/m² of an acryl-based resin was added to a polyester fiber (basis weight of 25 g/m², Eltas E01025 produced by Asahi Chemical Industry). The basis weight of thus obtained nonwoven fabric was 40 g/m².

Meanwhile, a monomer mixture including 95 parts of 2-ethylhexyl acrylate and 5 parts of acrylic acid was melted and mixed to 80 parts of ethyl acetate under a nitrogen gas atmosphere to be homogeneous. Thereto, 0.2 parts of benzoyl peroxide (BPO) was added as a polymerization initiating agent, and carried out a copolymerization reaction to obtain an acryl-based copolymer.

This acryl-based copolymer was coated on a silicone treated surface of a release sheet, in which the one surface had been subjected to a silicone treatment, to give a dry thickness of 40 µm. The coated sheet was dried for 3 minutes on a drying tower heated to 120 °C, and thus formed a crosslinked adhesive layer.

Next, the formed adhesive layer was adhered on the formed nonwoven fabric, and prepared a laminate having a pressure-sensitive adhesive layer on a substrate.

On the adhesive laminate having the pressure-sensitive adhesive layer, pores were formed. That is, pores were formed on the laminated as shown in Fig. 1A by using a punch roll having anchored projections. In specific, a punch roll equipping an abacus beads-shaped anchored projection (cross width of anchored projection (Y') was 0.98 mm, length width of anchored projection (X') was 0.61 mm, distance between anchored projections in a cross direction (N') was 0.31 mm, and distance between anchored projections in a longitudinal direction (M') was 5.0 mm) was set to a temperature of 290°C, and a metal flat roll having a smooth surface was set to a temperature of 100°C, to pass the nonwoven fabric through the punch roll and the metal flat roll under pressure with a punching stress of 33 kgf/cm and at a rate of 20 m/min. Accordingly, a pressure-sensitive adhesive tape with penetrating pores each having the size of 0.56 mm length width X and 0.63 mm cross width Y was prepared.

The obtained adhesive tape was subjected to a hand tearing ability evaluation. The results are shown in Table 1.

### Examples 2 and 3

Adhesive tapes were prepared in the same manner as in the Example 1, except that conditions for the punching process were changed as shown in the Table 1. Thus obtained adhesive tapes were subjected to the measurement and evaluation as in the Example 1. The results are shown in Table 1.

### Comparative Examples 1 to 3

Adhesive tapes were prepared in the same manner as in the Example 1, except that a punch roll having conical projections is used, and a punching temperature, a punching stress, and a punching rate, were changed as shown in Table 1.

Thus obtained adhesive tapes were subjected to the measurement and evaluation as in the Example 1. The results are shown in Table 1.

**Table 1**

| | Punching process | | | | Tensile strength | | | Hand Tearing property |
|---|---|---|---|---|---|---|---|---|
| | ***1** | ***2** | ***3** | Penetrating pore shape | ***4** | ***5** | ***6** | ***7** |
| E1 | 290 | 33 | 20 | flat transformed-hexagon | 15.4 | 10.8 | 1.43 | 0.9 |
| E2 | 290 | 23 | 30 | flat transformed-hexagon | 16.3 | 11.9 | 1.37 | 1.2 |
| E3 | 330 | 13 | 30 | flat transformed-hexagon | 11.5 | 11.8 | 0.97 | 1.0 |
| CE 1 | 290 | 33 | 30 | circular | 18.2 | 12.4 | 1.47 | 9.5 |
| CE 2 | 290 | 13 | 10 | circular | 16.4 | 11.6 | 1.41 | 9.7 |
| CE 3 | 290 | 52 | 30 | circular | 15.8 | 13.5 | 1.17 | 7.1 |

| | | | | | | | | |
|---|---|---|---|---|---|---|---|---|
| *1: Punch roll temperature (°C) *2: Punching stress (kgf/cm) *3: Punching rate (m/min) *4: Longitudinal strength (N/12mm) *5: Cross strength (N/12mm) *6: Strength ratio (longitudinal : cross) *7: Longest length (P) (mm) E: Example CE: Comparative example | | | | | | | | |

As clear from the Table 1, it was understood that the pressure-sensitive adhesive tapes of Examples 1 to 3 have an excellent tensile strength and an excellent hand tearing property.

On the other hand, it was understood that the pressure-sensitive adhesive tapes of Comparative Examples 1 to 3 have a hand tearing property of 7.1 mm or more such that the hand tearing property was inferior.

That is, it was understood that a pressure-sensitive adhesive tape causing less skin irritation and having excellent tensile strength and hand tearing property can be realized in accordance with the invention.

Next, examples related to the second embodiment of the invention will be described below.

In the present Examples and Comparative Examples, adhesive sheets were prepared by using the following materials.

As a nonwoven fabric constituting a substrate, a nonwoven fabric (basis weight of 25 g/m²) formed with a polyester-based fiber prepared by adding 25 g/m² of an acryl-based resin was used. The basis weight of thus obtained nonwoven fabric was 50 g/m².

For forming a pressure-sensitive adhesive layer, a monomer mixture as a polymer which includes 95 parts of 2-ethylhexyl acrylate and 5 parts of acrylic acid, 2 parts of sodium lauryl sulfate as an emulsifier, and 0.2 parts of potassium persulfate as a polymerization initiating agent, were used to conduct an emulsion polymerization by a well known method, and an acryl emulsion copolymer having a solid concentrate of 50% by weight was obtained.

This acryl emulsion copolymer was coated on a treated surface of a release sheet which is treated with a silicone-based resin, to give a dry thickness of 40 µm. The coated sheet was dried for 3 minutes on a drying tower heated to 160 °C, and thus formed a crosslinked adhesive layer. Subsequently, the nonwoven fabric was adhered on the pressure-sensitive adhesive layer to prepare a pressure-sensitive adhesive sheet having no pores.

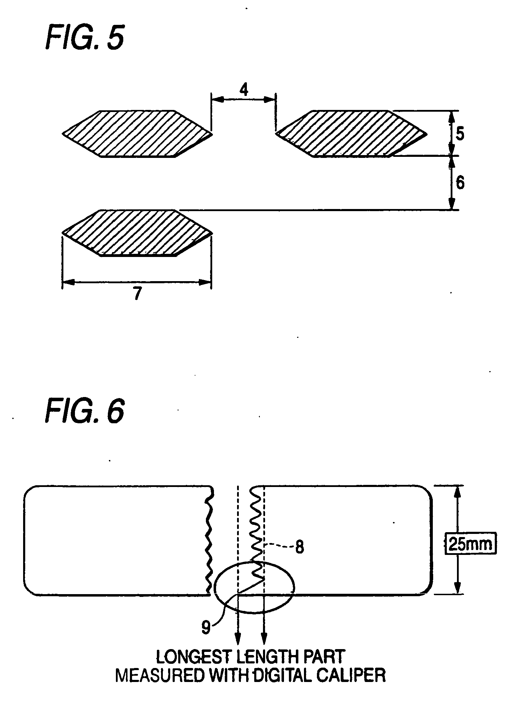

Next, on the prepared adhesive sheet, pores were formed under conditions shown in Table 2. The punch roll having hexagonal projections as shown in Fig. 5 was used. The projection size of the punch roll was cross width of 0.90 mm, length width of 0.60 mm, cross interval of 0.40 mm, and longitudinal interval of 5.0 mm.

Thus obtained adhesive sheet was first subjected to a penetration ratio measurement for the pores. The penetration ratio of the pores were measured by using microscope VQ-Z50 manufactured by KEYENCE Corporation. The pressure-sensitive adhesive sheet was magnified by 10 folds, and the number of penetrating pores and non-penetrating pores when the measurement was done 15 times at a 25 mm interval in one direction was measured. In the above manner, the penetration ratio was obtained from a ratio of the total number of penetrating pores and non-penetrating pores to the number of penetrating pores. The penetration ratio in the range of 50 to 100% was assumed as Examples and the ratio below 50% was assumed as Comparative Examples.

**Table 2**

| | Punching process | | | Punching perforation measurement | Pore Size | | |
|---|---|---|---|---|---|---|---|
| | *1 | *2 | *3 | *4 | *5 | *6 | *7 |
| E4 | 30 | 290 | 13 | 58 | 0.51 | 0.73 | 0.61 |
| E 5 | 30 | 300 | 13 | 70 | 0.48 | 0.72 | 0.57 |
| E6 | 30 | 310 | 13 | 83 | 0.48 | 0.68 | 0.61 |
| E7 | 30 | 320 | 13 | 83 | 0.48 | 0.69 | 0.61 |
| E8 | 23 | 310 | 13 | 100 | 0.50 | 0.61 | 0.67 |
| CE4 | 30 | 250 | 13 | 7 | 0.49 | 0.81 | 0.52 |
| CE 5 | 23 | 270 | 13 | 13 | 0.44 | 0.84 | 0.52 |
| CE6 | 23 | 290 | 13 | 40 | 0.45 | 0.78 | 0.60 |

| | | | | | | | |
|---|---|---|---|---|---|---|---|
| *1: Punching rate [m/min] *2: Punching R/L surface temperature [°C] *3: Punching stress [kgf/cm²] *4: Punching penetration ratio [%] *5: Longitudinal length of short side [mm] *6: Cross length of interval [mm] *7: Longitudinal length of long side [mm] E: Example CE: Comparative example | | | | | | | |

### TEST 1 Longest Cut Surface Measurement

The longest cut surface in adhesive sheets prepared in Examples and Comparative Examples were measured.

Each adhesive sheet was torn with fingers in the length of 25 mm in the direction (width direction) perpendicular to a machine direction. The torn direction was assumed as an ideal cut surface, and a distance between a cut line of the ideal cut surface and a most distanced cut edge was obtained (See Fig. 6). For example, when the tape was torn in a machine direction, a longest length among the lengths up to the cut edge of the pressure-sensitive adhesive sheet stretched in a direction perpendicular to a machine direction was measured. As the length was shorter, the hand tearing property was excellent. The aforementioned operation was repeated for 5 times, and the average value thereof was obtained, to find a longest cut surface. The length was measured with the use of Mitutoyo Digital Caliper CD-20, and values are shown in the Table 2.

### TEST 2 Tensile Strength Test

Each adhesive sheet obtained in Examples and Comparative Examples was subjected to a longitudinal tensile strength test. The test was carried out by measuring the longitudinal tensile strength at an extension rate of 300 mm/min under conditions of a sample measuring width of 25 mm and a sample measuring distance of 100 mm with the use of an autograph AG-IS manufactured by Shimazu Corporation. The numerical values were obtained in terms of a longitudinal tensile strength at a width of 12 mm, and the values are shown in the Table 2. The smaller numerical value indicates the easy cutting with a little force, and can be evaluated as an excellent hand tearing property.

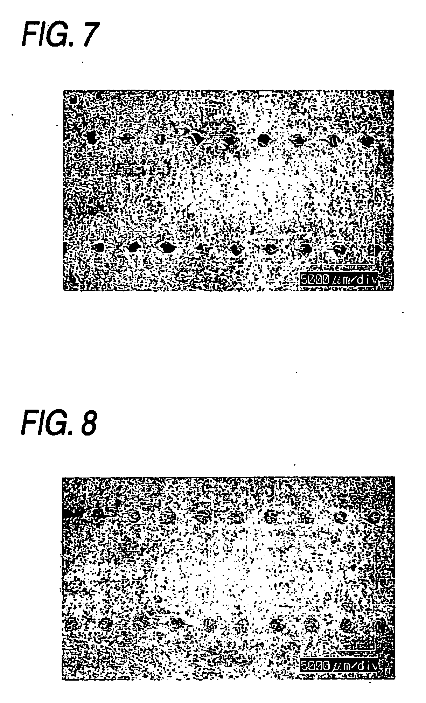

The results of longest cut surface and the tensile strength test for the pressure-sensitive adhesive sheets prepared in Examples and Comparative Examples were shown in Table 3. From the results, adhesive sheets of Examples 4 to 8 were confirmed to have an excellent hand tearing property, but effective hand tearing property was not obtained in the pressure-sensitive adhesive sheets of Comparative Examples 4 to 6. Pores provided on adhesive sheets obtained under conditions of Example 4 and Comparative Example 4 were shown in Fig. 7 and Fig. 8.

**Table 3**

| | **Test 1** | **Test 2** |
|---|---|---|
| | longest length of cut surface | extension test |
| | longest length of cut surface [mm] | Longitudinal extension strength [N/12mm] |
| **E4** | **1.5** | **15.6** |
| **E5** | **1.2** | **9.8** |
| **E6** | **1.2** | **6.0** |
| **E7** | **1.1** | **6.3** |
| **E8** | **0.6** | **9.3** |
| **CE 4** | **16.8** | **22.9** |
| **CE5** | **11.8** | **28.2** |
| **CE6** | **7.2** | **20.8** |

Further, examples which do not form part of the invention but are useful for the understanding of the invention will be described below.

### Example 9

A polyester fiber (basis weight of 25 g/m², Eltas E01025 produced by Asahi Chemical Industry) was impregnated with a chemical binder (water-based binder containing an acryl-based resin) and subjected to a binding treatment, to incorporate 15 g/m² of an acryl-based resin. The basis weight of the obtained nonwoven substrate was 40 g/m².

A mixture including 95 parts by weight of 2-ethylhexyl acrylate and 5 parts by weight of acrylic acid was mixed to ethyl acetate, 0.2 parts of benzoyl peroxide (BPO) was added as a polymerization initiating agent thereto, and carried out a copolymerization reaction under a nitrogen atmosphere to obtain an acryl-based copolymer. The obtained acryl-based copolymer was coated on a silicone treated surface of a release sheet which had been subjected to a silicone treatment, to give a dry thickness of 40 µm. The coated sheet was dried at 12 m/min on a drying tower heated to 120°C, and thus formed a pressure-sensitive adhesive layer. The pressure-sensitive adhesive layer was adhered with the obtained nonwoven substrate to prepare a pressure-sensitive adhesive sheet. On the pressure-sensitive adhesive sheet, pores were formed. That is, the pressure-sensitive adhesive sheet was cut to give a pressure-sensitive adhesive sheet having the size of about 50 cm in length and about 25 mm in width, and a penetration with 5 mm width (alignment interval of 5 mm) in a lengthwise direction was formed by using a cutter knife having an uneven round blade (depth of recessed part of 0.6 mm, height of projected part of 1.0 mm).

Four persons (I, II, III, IV) carried out the following evaluations on the obtained medical pressure-sensitive adhesive sheet, respectively. The results are shown in Table 4.

### Evaluation of Hand Tearing Property

The medical pressure-sensitive adhesive sheet was torn with fingers at a position of desired length in the length of 25 mm in the direction (width direction) perpendicular to a machine direction of the sheet. The measurement was carried out for three times (n = 3). The cut part of the medical pressure-sensitive adhesive sheet torn was observed, and occurrence of obtaining one-way cut surface was counted. The evaluation was carried out in accordance with the following evaluation standards.

### Evaluation Standard

A no deformation on edges, and straightly cut
B part held with fingers was deformed, but straightly cut
C part held with fingers was creased, and diagonally cut
D unable to cut

### Example 10

A medical pressure-sensitive adhesive sheet was prepared in the same manner as in the Example 9, except that the alignment interval for the penetration formed on a pressure-sensitive adhesive sheet was changed from 5 mm to 1 mm.

Thus obtained skin sticking adhesive sheet was evaluated as in the Example 9. The results are shown in Table 4.

### Example 11

A medical pressure-sensitive adhesive sheet was prepared in the same manner as in the Example 9, except that the alignment interval for the penetration formed on a pressure-sensitive adhesive sheet was changed from 5 mm to 3 mm.

Thus obtained skin sticking adhesive sheet was evaluated as in the Example 9. The results are shown in Table 4.

### Example 12

A medical pressure-sensitive adhesive sheet was prepared in the same manner as in the Example 9, except that the alignment interval for the penetration formed on a pressure-sensitive adhesive sheet was changed from 5 mm to 10 mm.

Thus obtained skin sticking adhesive sheet was evaluated as in the Example 9. The results are shown in Table 4.

### Comparative Example 7

A medical pressure-sensitive adhesive sheet was prepared in the same manner as in the Example 9, except that the alignment interval for the penetration formed on a pressure-sensitive adhesive sheet was changed from 5 mm to 17 mm.

Thus obtained skin sticking adhesive sheet was evaluated as in the Example 9. The results are shown in Table 4.

### Comparative Example 8

A medical pressure-sensitive adhesive sheet was prepared in the same manner as in the Example 9, except that the alignment interval for the pe formed on a pressure-sensitive adhesive sheet was changed from 5 mm to 20 mm.

Thus obtained skin sticking adhesive sheet was evaluated as in the Example 9. The results are shown in Table 4.

**Table 4**

| | **Alignment interval (mm)** | **I** | | **II** | | **III** | | **IV** | |
|---|---|---|---|---|---|---|---|---|---|
| | | ***1** | ***2** | ***1** | ***2** | ***1** | ***2** | ***1** | ***2** |
| **E9** | **5** | **3** | **A** | **3** | **A** | **3** | **A** | **3** | **A** |
| **E 10** | **1** | **3** | **A** | **3** | **A** | **3** | **A** | **3** | **A** |
| **E11** | **3** | **3** | **A** | **3** | **A** | **3** | **A** | **3** | **A** |
| **E12** | **10** | **3** | **A** | **3** | **A** | **3** | **A** | **3** | **A** |
| **CE7** | **17** | **0** | **D** | **0** | **D** | **3** | **B** | **1** | **D** |
| **CE 8** | **20** | **1** | **C** | **2** | **C** | **3** | **B** | **2** | **C** |

As clear from the Table 4, each of four persons (I, II, III, IV) was able to cut the medical pressure-sensitive adhesive tapes of Examples 9 to 12 in a desired length by hand, and additionally the straight cutting was achieved. Further, the medical pressure-sensitive adhesive tapes of Examples 9 to 12 were cut without giving an uneven cut edge. That is, it was realized that the medical pressure-sensitive adhesive tape of the invention is excellent in hand tearing property.

On the other hand, the tapes of Comparative Examples 7 and 8 having a pored part with more than 15 mm of alignment interval either caused a crease on the part held with fingers or a diagonal cut, and some people could not cut the tape at all.

The pressure-sensitive adhesive tapes with 0.5 mm of alignment interval some caused the cut surface to cut across the adjacent alignment of pores, thus hardly obtained a straight cut surface.

The pressure-sensitive adhesive tape or sheet of the invention can be used in the form of sheet or tape of various sizes, and also can be kept in a roll form. The pressure-sensitive adhesive tape or sheet can be used for purpose of sticking on the skin, and in the fields of hygiene in clinical practice and external use. In specific, the invention is preferably used for a plaster, an adhesive bandage, a dressing material, a tape for taping, or the like.

In addition, the invention provides a pressure-sensitive adhesive tape or sheet excellent in hand tearing property. Moreover, since the pressure-sensitive adhesive tape or sheet of the invention has a substrate including nonwoven fabric and a pressure-sensitive adhesive layer on at least one side of the substrate, it provides a pleasant texture and is preferably used in the clinical field. In particular, since the pressure-sensitive adhesive tape or sheet of the invention has penetrating pores, it is preferably used as a pressure-sensitive adhesive tape or sheet for a clinical use as it is kind to skin by eliminating the cause for dampness and irritation, in addition to the provision of excellent hand tearing property.

Further, the pressure-sensitive adhesive tape or sheet of the invention can be preferably used as a pressure-sensitive adhesive tape or sheet useful in the clinical field, sport field, etc, and particularly preferably used when a hand tearing property is demanded. It can also be used, for example, as an emergency adhesive plaster or plaster, an adhesive bandage, and a tape for taping, by being cut in an appropriate size or shape according to the necessity.

While the present invention has been described in detail and with reference to specific embodiments thereof, it will be apparent to one skilled in the art that various changes and modifications can be made therein without departing from the scope thereof.

This application is based on Japanese patent application No. 2006-065780 filed March 10, 2006, Japanese patent application No. 2006-111389 filed April 13, 2006 and Japanese patent application No. 2006-163421 filed June 13, 2006.

## Claims

1. A pressure-sensitive adhesive tape or sheet (A) comprising:
a substrate comprising a nonwoven fabric; and
a pressure-sensitive adhesive layer disposed on at least one side of the substrate,
said pressure-sensitive adhesive tape or sheet (A) having a plurality of penetrating pores (1), **characterized in that** said penetrating pores (1) penetrate the substrate and the pressure-sensitive adhesive layer so that corners of said penetrating pores (1) are aligned in at least one direction,
said penetrating pores (1) having a flat polygonal shape, and
wherein said penetration pore has, among the corners aligned in one direction, at least two corners facing each other in one direction each having an angle (α,β) of less than 90°.

2. The pressure-sensitive adhesive tape or sheet according to claim 1, wherein the flat polygonal shape of said penetrating pore (1) is a flat hexagonal shape or a flat transformed-hexagonal shape.

3. The pressure-sensitive adhesive tape or sheet according to claim 1, wherein said penetrating pore (1) is formed by punching with an abacus bead-shaped metal mold.

4. The pressure-sensitive adhesive tape or sheet according to claim 1, wherein a distance (4) between the corner of one penetrating pore (1) having an angle (α,β) of less than 90° and the corner of another penetrating pore adjacent thereto having an angle of less than 90°, said penetrating pores (1) being aligned, is in the range of 0.3 mm to 3 mm.

5. The pressure-sensitive adhesive tape or sheet according to claim 1, wherein a distance (N) between one penetrating pore and another penetrating pore adjacent thereto, said penetrating pores (1) being aligned, is equal to or longer than a length of a flattened part of the penetrating pore (1).

6. The pressure-sensitive adhesive tape or sheet according to claim 1, wherein the nonwoven fabric is formed by a thermoplastic resin.

7. The pressure-sensitive adhesive tape or sheet according to claim 1, wherein the substrate comprises a binder impregnated therein.

8. The pressure-sensitive adhesive tape or sheet according to claim 1, which is subjected to a peeling treatment on the side of the substrate opposite to the side on which the pressure-sensitive adhesive layer is disposed.

9. The pressure-sensitive adhesive tape or sheet according to claim 1, which is wound in a roll form for keeping.

10. A pressure-sensitive adhesive tape or sheet (A) comprising:
a substrate comprising a nonwoven fabric; and
a pressure-sensitive adhesive layer disposed on at least one side of the substrate,
said pressure-sensitive adhesive tape or sheet having a plurality of pores (1) aligned in at least one direction,
**characterized by**
said pores having a shape in which at least two corners facing each other in one direction each has an angle (α,β) of less than 90°,
said pores (1) including penetrating and non-penetrating pores,
said pressure-sensitive adhesive tape or sheet (A) having a penetration ratio of a series of adjacent pores (1) of 50% or more and less than 100%, and
wherein the penetrating and non-penetrating pores (1) are disposed randomly.

11. The pressure-sensitive adhesive tape or sheet according to claim 10, wherein a distance (4) between one pore (1) and another pore (1) adjacent thereto among pores (1) aligned in at least one direction is in the range of 0.3 to 0.75 mm.

12. The pressure-sensitive adhesive tape or sheet according to claim 10, wherein the pore (1) has a length of long-sided part in the range of 0.3 to 0.75 mm.

13. The pressure-sensitive adhesive tape or sheet according to claim 10, wherein the penetrating pore (1) has a size in the range of 1 x 10⁻⁴ to 3 mm².

14. The pressure-sensitive adhesive tape or sheet according to claim 10, wherein the nonwoven fabric is formed by a thermoplastic resin.

## Patentansprüche

1. Druckempfindliches Klebeband bzw. druckempfindliche Klebebahn (A), das/die umfasst:
ein Substrat, das einen Vliesstoff umfasst; und
eine Schicht aus druckempfindlichem Klebstoff, die an wenigstens einer Seite des Substrats angeordnet ist,
wobei das druckempfindliche Klebeband bzw. die druckempfindliche Klebebahn (A) eine Vielzahl von Durchgangsporen (1) aufweist, **dadurch gekennzeichnet, dass** die Durchgangsporen (1) durch das Substrat und die Schicht aus druckempfindlichem Klebstoff so hindurch verlaufen, dass Ecken der Durchgangsporen (1) in wenigstens einer Richtung fluchtend sind,
die Durchgangsporen (1) eine flache Vieleckform haben, und
wobei die Durchgangspore von den in einer Richtung fluchtenden Ecken wenigstens zwei Ecken hat, die einander in einer Richtung zugewandt sind und jeweils einen Winkel (α, β) von weniger als 90° haben.

2. Druckempfindliches Klebeband oder druckempfindliche Klebebahn nach Anspruch 1, wobei die flache Vieleckform der Durchgangspore (1) eine flache Sechseckform oder eine flache transformierte Sechseckform ist.

3. Druckempfindliches Klebeband oder druckempfindliche Klebebahn nach Anspruch 1, wobei die Durchgangspore (1) durch Stanzen mit einem Metallwerkzeug in Form eines Abakus-Kügelchens ausgebildet wird.

4. Druckempfindliches Klebeband oder druckempfindliche Klebebahn nach Anspruch 1, wobei ein Abstand (4) zwischen der Ecke einer Durchgangspore (1), die einen Winkel (α, β) von weniger als 90° hat, und der Ecke einer anderen, daran angrenzenden Durchgangspore, die einen Winkel von weniger als 90° hat, wenn die Durchgangsporen (1) fluchtend sind, im Bereich von 0,3 mm bis 3 mm liegt.

5. Druckempfindliches Klebeband oder druckempfindliche Klebebahn nach Anspruch 1, wobei ein Abstand (N) zwischen einer Durchgangspore und einer daran angrenzenden anderen Durchgangspore, wenn die Durchgangsporen (1) fluchtend sind, genauso groß ist wie oder größer als die Länge eines abgeflachten Teils der Durchgangspore (1).

6. Druckempfindliches Klebeband oder druckempfindliche Klebebahn nach Anspruch 1, wobei der Vliesstoff mit einem thermoplastischem Harz hergestellt wird.

7. Druckempfindliches Klebeband oder druckempfindliche Klebebahn nach Anspruch 1, wobei das Substrat ein Bindemittel umfasst, mit dem es imprägniert ist.

8. Druckempfindliches Klebeband oder druckempfindliche Klebebahn nach Anspruch 1, die einer Abziehbehandlung an der Seite des Substrats unterzogen wird, die der Seite gegenüberliegt, an der sich die Schicht aus druckempfindlichem Klebstoff befindet.

9. Druckempfindliches Klebeband oder druckempfindliche Klebebahn nach Anspruch 1, das/die zur Aufbewahrung in einer Rollenform aufgewickelt ist.

10. Druckempfindliches Klebeband oder druckempfindliche Klebebahn (A), das/die umfasst:
ein Substrat, das einen Vliesstoff umfasst; und
eine Schicht aus druckempfindlichem Klebstoff, die an wenigstens einer Seite des Substrats angeordnet ist,
wobei das druckempfindliche Klebeband bzw. die druckempfindliche Klebebahn eine Vielzahl von Poren (1) aufweist, die in wenigstens einer Richtung fluchtend sind,
**dadurch gekennzeichnet, dass**
die Poren eine Form haben, in der wenigstens zwei Ecken, die einander in einer Richtung zugewandt sind, jeweils einen Winkel (α,β) von weniger als 90° haben,
die Poren (1) Durchgangs- und Nicht-Durchgangs-Poren einschließen,
das druckempfindliche Klebeband bzw. die druckempfindliche Klebebahn (A) ein Durchdringungsverhältnis einer Reihe benachbarter Poren (1) von 50 % oder mehr und weniger als 100 % hat, und
wobei die Durchgangs- und Nicht-Durchgangs-Poren (1) willkürlich angeordnet sind.

11. Druckempfindliches Klebeband oder druckempfindliche Klebebahn nach Anspruch 10, wobei ein Abstand (4) zwischen einer Pore (1) und einer daran angrenzenden anderen Pore (1) von in wenigstens einer Richtung fluchtenden Poren (1) im Bereich von 0,3 bis 0,75 mm liegt.

12. Druckempfindliches Klebeband oder druckempfindliche Klebebahn nach Anspruch 10, wobei die Pore (1) eine Länge eines Teils an der langen Seite im Bereich von 0,3 bis 0,75 mm hat.

13. Druckempfindliches Klebeband oder druckempfindliche Klebebahn nach Anspruch 10, wobei die Durchgangs-Pore (1) eine Größe im Bereich 1 x 10⁻⁴ bis 3 mm² hat.

14. Druckempfindliches Klebeband oder druckempfindliche Klebebahn nach Anspruch 10, wobei der Vliesstoff mit einem thermoplastischem Harz hergestellt wird.

## Revendications

1. Bande ou feuille adhésive autocollante (A), comprenant :
un substrat comprenant un non-tissé, et
une couche adhésive autocollante disposée sur au moins une face du substrat,
ladite bande ou feuille adhésive autocollante (A) ayant une série de pores traversants (1), **caractérisée en ce que** lesdits pores traversants (1) percent le substrat et la couche adhésive autocollante de sorte que les coins desdits pores traversants (1) sont alignés selon au moins une direction,
lesdits pores traversants (1) ayant une forme polygonale plane, et
où ledit pore traversant (1) a, parmi les coins alignés selon un direction, au moins deux coins se faisant face selon une direction, chacun ayant un angle (α, β) inférieur à 90°.

2. Bande ou feuille adhésive autocollante selon la revendication 1, où la forme polygonale plane dudit pore traversant (1) est une forme hexagonale plane ou une forme hexagonale transformée plane.

3. Bande ou feuille adhésive autocollante selon la revendication 1, où ledit pore pénétrant (1) est formé par percement avec une matrice métallique en forme de boulier.

4. Bande ou feuille adhésive autocollante selon la revendication 1, où la distance (4) entre le coin d'un pore traversant (1) ayant un angle (α, β) inférieur à 90° et le coin d'un autre pore traversant adjacent ayant un angle inférieur à 90°, lesdits pores traversants (1) étant alignés, se situe dans l'intervalle allant de 0,3 mm à 3 mm.

5. Bande ou feuille adhésive autocollante selon la revendication 1, où la distance (N) entre un pore traversant et un autre pore traversant adjacent, lesdits pores traversants (1) étant alignés, est égale ou supérieure à la longueur d'une partie plane du pore traversant (1).

6. Bande ou feuille adhésive autocollante selon la revendication 1, où le non-tissé est formé d'une résine thermoplastique.

7. Bande ou feuille adhésive autocollante selon la revendication 1, où le substrat comprend un liant, imprégné dans celui-ci.

8. Bande ou feuille adhésive autocollante selon la revendication 1, qui est soumis à un traitement de pelage sur la face du substrat, opposée à la face sur laquelle la couche adhésive autocollante est disposée.

9. Bande ou feuille adhésive autocollante selon la revendication 1, qui est enroulée sous forme d'un rouleau pour la conservation.

10. Bande ou feuille adhésive autocollante (A), comprenant :
un substrat comprenant un non-tissé, et
une couche adhésive autocollante disposée sur au moins une face du substrat,
ladite bande ou feuille adhésive autocollante ayant une série de pores (1) alignés selon au moins une direction, **caractérisée en ce que**
lesdits pores (1) ont une forme dans laquelle au moins deux coins se faisant face selon une direction ont chacun un angle (α, β) inférieur à 90°,
lesdits pores (1) comprennent des pores traversants et des pores non traversants,
ladite bande ou feuille adhésive autocollante (A) a un rapport de pénétration d'une série de pores adjacents (1) de 50% ou plus et inférieur à 100%, et
où les pores traversants et les pores non traversants (1) sont disposés au hasard.

11. Bande ou feuille adhésive autocollante selon la revendication 10, où la distance (4) entre un pore (1) et un autre pore (1) adjacent, parmi les pores (1) alignés selon au moins une direction, se situe dans l'intervalle allant de 0,3 mm à 0,75 mm.

12. Bande ou feuille adhésive autocollante selon la revendication 10, où le pore (1) a une longueur d'un long côté, située dans l'intervalle allant de 0,3 à 0,75 mm.

13. Bande ou feuille adhésive autocollante selon la revendication 10, où le pore traversant (1) a une taille située dans l'intervalle allant de 1 x 10⁻⁴ à 3 mm².

14. Bande ou feuille adhésive autocollante selon la revendication 10, où le non-tissé est formé d'une résine thermoplastique.
